(19) **Europäisches Patentamt / European Patent Office / Office européen des brevets**

(11) **EP 4 010 685 B1**

(12) **FASCICULE DE BREVET EUROPEEN**

(45) Date de publication et mention
de la délivrance du brevet:
**30.10.2024 Bulletin 2024/44**

(21) Numéro de dépôt: **20749839.5**

(22) Date de dépôt: **28.07.2020**

(51) Classification Internationale des Brevets (IPC):
***G01N 21/27*** *(2006.01)* ***G01N 21/31*** *(2006.01)*
***G01N 21/3504*** *(2014.01)* ***G01N 33/00*** *(2006.01)*

(52) Classification Coopérative des Brevets (CPC):
**G01N 33/0006; G01N 21/274; G01N 21/314;**
**G01N 21/3504;** G01N 2021/3155;
G01N 2021/3166; G01N 2021/3177

(86) Numéro de dépôt international:
**PCT/EP2020/071251**

(87) Numéro de publication internationale:
**WO 2021/023576 (11.02.2021 Gazette 2021/06)**

(54) **PROCÉDÉ D'ANALYSE D'UN GAZ PAR UN CAPTEUR OPTIQUE**

VERFAHREN ZUR ANALYSE EINES GASES MITTELS EINES OPTISCHEN SENSORS

METHOD FOR ANALYSING A GAS USING AN OPTICAL SENSOR

(84) Etats contractants désignés:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priorité: **06.08.2019 FR 1909028**

(43) Date de publication de la demande:
**15.06.2022 Bulletin 2022/24**

(73) Titulaire: **Elichens**
**38040 Grenoble (FR)**

(72) Inventeur: **LE, Than Trung**
**décédé (FR)**

(74) Mandataire: **INNOV-GROUP**
**209 Avenue Berthelot**
**69007 Lyon (FR)**

(56) Documents cités:
**DE-A1- 19 925 196 FR-A1- 3 077 387**
**US-A1- 2015 338 339 US-A1- 2017 038 354**
**US-B1- 6 191 421**

**Description**

**DOMAINE TECHNIQUE**

Le domaine technique de l'invention est un procédé optique d'analyse d'un gaz, en mettant en oeuvre une source de lumière de type corps noir ou corps gris et en mesurant une absorption d'une onde lumineuse émise par la source de lumière.

**ART ANTERIEUR**

Le recours à des méthodes optiques pour l'analyse d'un gaz est fréquent. Des capteurs permettent de déterminer la composition d'un gaz en se basant sur le fait que les espèces composant le gaz présentent des propriétés spectrales d'absorption différentes les unes des autres. Ainsi, connaissant une bande spectrale d'absorption d'une espèce gazeuse, sa concentration peut être déterminée par une estimation de l'absorption de la lumière traversant le gaz, en utilisant la loi de Beer-Lambert. Ce principe permet une estimation de la concentration d'une espèce gazeuse présente dans le gaz.

Selon les procédés les plus courants, le gaz analysé s'étend entre une source de lumière et un photodétecteur, dit photodétecteur de mesure, ce dernier étant destiné à mesurer une onde lumineuse transmise par le gaz à analyser, l'onde lumineuse étant partiellement absorbée par ce dernier. La source de lumière est usuellement une source émettant dans l'infrarouge, la méthode utilisée étant usuellement désignée par le terme anglosaxon "NDIR détection", l'acronyme NDIR signifiant Non Dispersive Infra-Red. Un tel principe a été fréquemment mis en oeuvre, et est par exemple décrit dans les documents US5026992 ou WO2007064370.

Les procédés usuels comprennent généralement une mesure d'une onde lumineuse, dite onde lumineuse de référence, émise par la source, l'onde lumineuse de référence étant non absorbée, ou absorbée de façon négligeable, par le gaz analysé. La mesure de l'onde lumineuse de référence permet d'estimer l'intensité de l'onde lumineuse émise par la source, ou d'estimer l'onde lumineuse qui serait détectée par le photodétecteur de mesure en l'absence d'absorption par le gaz analysé. Cette technologie est désignée par le terme "double beam". La comparaison entre l'onde lumineuse en présence de gaz et l'onde lumineuse sans gaz permet de caractériser l'absorption du gaz. Il s'agit par exemple de déterminer une quantité d'une espèce gazeuse dans le gaz, selon la technologie désignée par le terme "NDIR par absorption".

L'onde lumineuse de référence est mesurée par un photodétecteur de référence. Il peut s'agir d'un photodétecteur de référence différent du photodétecteur de mesure, et agencé de façon à être disposé face à la source de lumière, le photodétecteur de référence étant associé à un filtre optique de référence. Le filtre optique de référence définit une bande spectrale de référence, dans laquelle le gaz à analyser ne présente pas d'absorption significative.

Selon une approche, décrite dans US2011/0042570, on utilise un photodétecteur de mesure et un photodétecteur de référence, les deux photodétecteurs détectant une onde lumineuse dans une même bande spectrale, en l'occurrence une bande spectrale d'absorption du $CO_2$. Le photodétecteur de référence est disposé plus proche de la source de lumière que le photodétecteur de mesure. La comparaison des signaux respectivement mesurés par le photodétecteur de mesure et le photodétecteur de référence permet de s'affranchir de la connaissance de l'intensité de l'onde lumineuse émise par la source.

Le document FR3000548, ou le document US 2015/338339 A1, décrit un capteur de $CO_2$ comportant une voie de mesure, dans une bande spectrale infrarouge, et une voie de référence, dans une bande spectrale visible (0.4 $\mu$m à 0.8 $\mu$m). La voie de référence est considérée comme non influencée par la concentration en $CO_2$ dans le gaz mesuré. Afin de tenir compte de l'évolution du spectre d'émission de la source de lumière, ce document décrit le recours à une fonction F, représentative du vieillissement de la source de lumière respectivement dans les bandes spectrales visible et infra-rouge. La fonction F est approximée par une fonction identité : ainsi, le vieillissement de la source de lumière dans l'infrarouge est considéré comme égal au vieillissement de la source de lumière dans le visible.

Le document US 6 191 421 81 concerne un instrument permettant de mesurer la concentration de dioxyde de carbone contenu dans un gaz respiratoire par une méthode infrarouge non dispersive. Le document FR 3 077 387 A1 concerne un procédé d'analyse d'un gaz, pour estimer une quantité de plusieurs espèces gazeuses différentes.

L'inventeur a constaté que le recours à une onde lumineuse de référence peut présenter certains inconvénients. Il propose un procédé permettant de surmonter ces inconvénients, de façon à améliorer la précision de la mesure.

**EXPOSE DE L'INVENTION**

Un premier objet de l'invention est un procédé de mesure d'une quantité d'une espèce gazeuse présente dans un gaz, l'espèce gazeuse étant apte à absorber une lumière dans une bande spectrale d'absorption le procédé comportant les étapes suivantes :

a) disposition du gaz entre une source de lumière et un photodétecteur de mesure, la source de lumière étant apte à émettre une onde lumineuse incidente, l'onde lumineuse incidente se propageant à travers le gaz vers le photodétecteur de mesure, la source de lumière étant parcourue par un courant électrique d'alimentation, de façon à porter la source de lumière à une valeur de température ;

b) illumination du gaz par la source de lumière;

c) mesure, par le photodétecteur de mesure, d'une intensité, dite intensité de mesure, d'une onde lumineuse transmise par le gaz, dans une bande spectrale de mesure, comportant la bande spectrale d'absorption;

d) mesure, par un photodétecteur de référence, d'une intensité, dite intensité de référence, d'une onde lumineuse de référence, l'onde lumineuse de référence étant émise par la source de lumière dans une bande spectrale de référence;

les étapes b) à d) étant mises en oeuvre à une pluralité d'instants de mesure, le procédé comportant, à chaque instant de mesure :

e) à partir de l'intensité de référence mesurée par le photodétecteur de référence, prise en compte d'une fonction de correction, représentative d'une variation d'une intensité de l'onde lumineuse incidente dans la bande spectrale de mesure relativement à une intensité de l'onde lumineuse incidente dans la bande spectrale de référence;

f) estimation d'une quantité de l'espèce gazeuse, à partir de l'intensité de mesure mesurée lors de l'étape c), de l'intensité de référence mesurée lors de l'étape d), et de la fonction de correction prise en compte lors de l'étape e) ;

le procédé étant caractérisé en ce que la fonction de correction est préalablement établie au cours d'une phase de calibration, en comparant, à différents niveaux de température, ou à différents niveaux du courant d'alimentation, des intensités lumineuses respectivement émises, par une source de lumière de test, dans la bande spectrale de mesure et dans la bande spectrale de référence, la source de lumière de test étant considérée comme représentative de la source de lumière mise en oeuvre dans l'étape b), à chaque instant de mesure.

Selon un mode de réalisation, la calibration est réalisée en utilisant une expression théorique définissant une intensité d'émission de la source de lumière de test en fonction de la température de la source de lumière et de la longueur d'onde, la calibration étant réalisée en considérant respectivement une longueur d'onde dans la bande spectrale de mesure et une longueur d'onde dans la bande spectrale de référence. La calibration peut comporter une détermination d'une fonction de vieillissement, reliant un vieillissement de la source de lumière, dans la bande spectrale de référence, à un vieillissement de la source de lumière, dans la bande spectrale de mesure. La fonction de correction est alors déterminée à partir de la fonction de vieillissement.

Selon un mode de réalisation, la calibration est réalisée de façon expérimentale, et comporte :

- disposition de la source de lumière de test, face à un photodétecteur de mesure de test et face à un photodétecteur de référence de test, le photodétecteur de mesure de test et le photodétecteur de référence de test étant respectivement représentatifs du photodétecteur de mesure et du photodétecteur de référence;
- illumination du photodétecteur de mesure de test et du photodétecteur de référence de test par la source de lumière de test, la source de lumière de test étant successivement portée à différents valeurs de température par différents courants d'alimentation;
- à chaque valeur de température, comparaison d'une intensité lumineuse détectée par le photodétecteur de mesure de test, dans la bande spectrale de mesure, avec une intensité lumineuse détectée par le photodétecteur de référence de test, dans la bande spectrale de référence, la fonction de correction étant établie à partir des comparaisons effectuées à chaque valeur de température.

La calibration peut comporter une détermination d'une fonction de vieillissement, reliant un vieillissement de la source de lumière, dans la bande spectrale de référence, à un vieillissement de la source de lumière, dans la bande spectrale de mesure, le procédé étant tel que la fonction de correction est déterminée à partir de la fonction de vieillissement.

La source de lumière de test peut être confondue avec la source de lumière mise en oeuvre dans l'étape b), à chaque instant de mesure.

Le procédé peut être tel que :

- l'étape e) comporte, à partir de la fonction de correction et de l'intensité mesurée par le photodétecteur de référence lors de l'étape d), une estimation d'une intensité qui serait détectée, par le photodétecteur de mesure, en l'absence de gaz ;

- lors de l'étape f), la quantité d'espèce gazeuse est déterminée à partir d'une comparaison entre l'intensité mesurée par le photodétecteur de mesure, lors de l'étape c), et l'intensité estimée lors de l'étape e).

Le procédé peut être tel que :

- l'étape e) comporte, à partir de la fonction de correction et de l'intensité mesurée par le photodétecteur de référence lors de l'étape d), une détermination d'une intensité corrigée, l'intensité corrigée correspondant à une intensité qui serait détectée, par le photodétecteur de mesure, en l'absence de vieillissement de la source de lumière;
- lors de l'étape f), la quantité d'espèce gazeuse est déterminée à partir d'une comparaison entre l'intensité corrigée résultant de l'étape e) et d'une estimation d'une intensité qui serait détectée par le photodétecteur de mesure, en l'absence de gaz entre la source de lumière et le photodétecteur de mesure, et en l'absence de vieillissement de la source de lumière.

Quel que soit le mode de réalisation, l'étape e) peut comporter une estimation d'un vieillissement de la source de lumière, dans la bande spectrale de référence, à partir de l'intensité de référence mesurée à l'instant de mesure, et de l'intensité de référence mesurée à un instant initial. Le vieillissement de la source de lumière, dans la bande spectrale de référence peut être calculé à partir d'une comparaison entre l'intensité de référence mesurée à l'instant de mesure et l'intensité de référence mesurée à l'instant initial. La comparaison peut par exemple prendre la forme d'une soustraction ou d'un ratio. La comparaison peut être normalisée par l'intensité de référence mesurée à l'instant initial.

Quel que soit le mode de réalisation, la calibration vise à établir une fonction de vieillissement, pour estimer un vieillissement de la source de lumière dans la bande spectrale de mesure à partir d'un vieillissement de la source de lumière dans la bande spectrale de référence. Le vieillissement, dans une bande spectrale, correspond à une comparaison entre une intensité mesurée, dans la bande spectrale, à l'instant de mesure avec une intensité mesurée, dans la bande spectrale, à un instant initial.

Selon un mode de réalisation, la source de lumière est un corps noir ou considérée comme étant un corps noir.

Un deuxième objet de l'invention est un dispositif pour déterminer une quantité d'une espèce gazeuse dans un gaz, le dispositif comportant :

- une source lumineuse configurée pour émettre une onde lumineuse incidente se propageant vers le gaz, l'onde lumineuse incidente s'étendant dans une bande spectrale d'absorption de l'espèce gazeuse ;
- un photodétecteur de mesure, configuré pour détecter une onde lumineuse transmise par le gaz, à différents instants de mesure, dans une bande spectrale de mesure et à en mesurer une intensité, dite intensité de mesure;
- un photodétecteur de référence, configuré pour mesurer une intensité, dite intensité de référence, d'une onde lumineuse de référence émise par la source de lumière, dans une bande spectrale de référence, aux différents instants de mesure ;
- un processeur, pour mettre en oeuvre les étapes e) et f) d'un procédé selon le premier objet de l'invention, à partir de l'intensité de référence et de l'intensité de mesure,

le dispositif étant caractérisé en ce que le processeur est configuré pour mettre en oeuvre une fonction de correction établie au cours d'une phase de calibration, en comparant, à différents niveaux de température, ou à différents niveaux du courant d'alimentation, des intensités lumineuses respectivement émises, par une source de lumière de test, dans la bande spectrale de mesure et dans la bande spectrale de référence, la source de lumière de test étant considérée comme représentative de la source de lumière mise en oeuvre dans l'étape b) d'un procédé selon le premier objet de l'invention, à chaque instant de mesure.

L'invention sera mieux comprise à la lecture de l'exposé des exemples de réalisation présentés, dans la suite de la description, en lien avec les figures listées ci-dessous.

## FIGURES

La figure 1A représente un exemple de dispositif permettant la mise en oeuvre de l'invention.
La figure 1B schématise un spectre d'émission d'une source de lumière de type corps noir.
La figure 2A montre la décroissance observée de l'intensité lumineuse émise par une source de lumière dans deux bandes spectrales différentes.
La figure 2B illustre la perte d'émissivité de la source de lumière dans une bande spectrale de mesure en fonction de la perte d'émissivité de la source de lumière dans une bande spectrale de référence. La figure 2B a été obtenue à partir d'une expression théorique de l'émissivité de la source de lumière en fonction de la température et de la longueur d'onde.

La figure 2C illustre une décroissance relative du signal détecté par un photodétecteur de mesure, dans une bande spectrale de mesure. La figure 2C a été obtenue à partir d'essais expérimentaux.

La figure 2D représente une relation entre une puissance d'un courant électrique alimentant une source de lumière et la température de la source de lumière.

La figure 3 représente les principales étapes d'un procédé mettant en oeuvre l'invention.

## EXPOSE DE MODES DE REALISATION PARTICULIERS

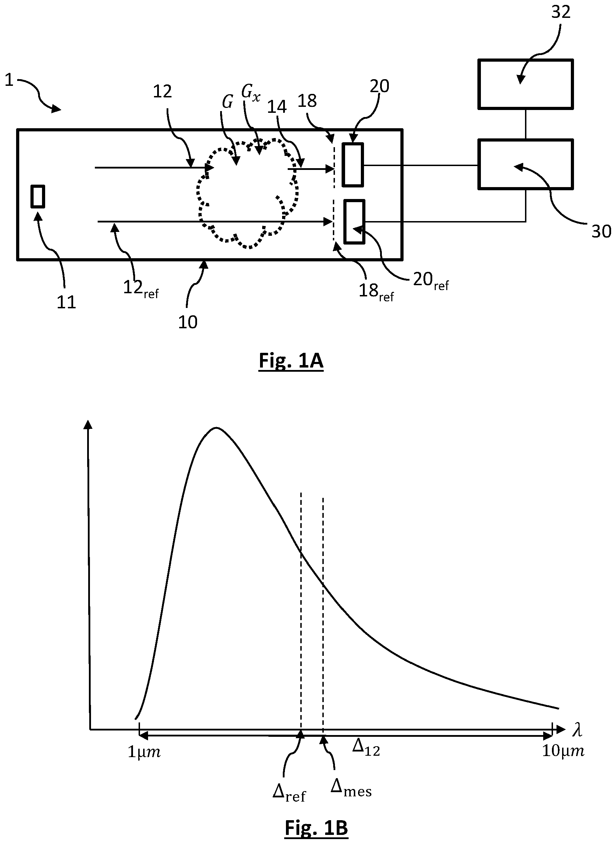

La figure 1A est un exemple d'un dispositif d'analyse 1 de gaz. Ce dispositif comporte une enceinte 10 définissant un espace interne à l'intérieur duquel se trouvent :

- une source de lumière 11, apte à émettre une onde lumineuse 12, dite onde lumineuse incidente, de façon à illuminer un gaz $G$ s'étendant dans l'espace interne. L'onde lumineuse incidente 12 s'étend selon une bande spectrale d'illumination $\Delta_{12}$.
- Un photodétecteur 20, dit photodétecteur de mesure, configuré pour détecter une onde lumineuse 14 transmise par le gaz $G$, sous l'effet de l'illumination de ce dernier par l'onde lumineuse incidente 12. L'onde lumineuse 14 est désignée par le terme onde lumineuse de mesure. Elle est détectée, par le photodétecteur de mesure 20, dans une bande spectrale de mesure $\Delta_{mes}$.
- Un photodétecteur de référence $20_{ref}$, configuré pour détecter une onde lumineuse $12_{ref}$, dite de référence dans une bande spectrale de référence $\Delta_{ref}$. La bande spectrale de référence $\Delta_{ref}$ est une bande spectrale dans laquelle on considère que l'absorption de l'onde lumineuse 12 par le gaz $G$ est négligeable.

La bande spectrale de référence $\Delta_{ref}$ est différente de la bande spectrale de mesure $\Delta_{mes}$. La bande spectrale de mesure $\Delta_{mes}$ peut notamment être plus large que la bande spectrale de référence $\Delta_{ref}$. La bande spectrale de mesure $\Delta_{mes}$ peut comprendre la bande spectrale de référence $\Delta_{ref}$.

Le gaz $G$ comporte une espèce gazeuse $G_x$ dont on cherche à déterminer une quantité $c_x(k)$, par exemple une concentration, à un instant de mesure $k$. Cette espèce gazeuse absorbe une part mesurable de la lumière dans une bande spectrale d'absorption $\Delta_x$.

La source de lumière 11 est apte à émettre l'onde lumineuse incidente 12, selon la bande spectrale d'illumination $\Delta_{12}$, cette dernière pouvant s'étendre entre le proche ultraviolet et l'infrarouge moyen, par exemple entre 200 nm et 10 $\mu$m, et le plus souvent entre 1 $\mu$m et 10 $\mu$m. La bande spectrale d'absorption $\Delta_x$ de l'espèce gazeuse $G_x$ analysée est comprise dans la bande spectrale d'illumination $\Delta_{12}$. La source de lumière 11 peut notamment être impulsionnelle, l'onde lumineuse incidente 12 étant une impulsion de durée généralement comprise entre 100 ms et 1 s. La source de lumière 11 peut notamment être une source de lumière de type filament suspendu et chauffé à une température comprise entre 400°C et 800°C. Son spectre d'émission, dans la bande spectrale d'illumination $\Delta_{12}$, correspond au spectre d'émission d'un corps noir.

Le photodétecteur de mesure 20 est de préférence associé à un filtre optique 18, définissant la bande spectrale de mesure $\Delta_{mes}$ englobant tout ou partie de la bande spectrale d'absorption $\Delta_x$ de l'espèce gazeuse.

Dans l'exemple considéré, le photodétecteur de mesure 20 est une thermopile, apte à délivrer un signal dépendant de l'intensité de l'onde lumineuse détectée. De façon alternative, le photodétecteur de mesure peut être une photodiode ou un autre type de photodétecteur.

Le photodétecteur de référence $20_{ref}$ est disposé à côté du photodétecteur de mesure 20 et est de même type que ce dernier. Il est associé à un filtre optique, dit filtre optique de référence $18_{ref}$. Le filtre optique de référence $18_{ref}$ définit la bande spectrale de référence $\Delta_{ref}$ correspondant à une plage de longueurs d'onde non absorbées par l'espèce gazeuse considérée. La bande passante de référence $\Delta_{ref}$ est par exemple centrée autour de la longueur d'onde 3.91 $\mu$m.

L'intensité $I(k)$ de l'onde lumineuse 14 détectée par le photodétecteur de mesure 20, dite intensité de mesure, à un instant de mesure $k$, dépend de la quantité $c_x(k)$ à l'instant de mesure, selon la relation de Beer-Lambert :

$$abs(k) = 1 - \frac{I(k)}{I_0(k)} = 1 - e^{-\mu(c_x(k))l} \quad (1)$$

où :

- $\mu(c_x(k))$ est un coefficient d'absorption, dépendant de la quantité $c_x(k)$ à l'instant $k$;
- $l$ est l'épaisseur de gaz traversée par l'onde lumineuse dans l'enceinte 10;
- $I_0(k)$ est l'intensité de l'onde lumineuse incidente, à l'instant $k$, qui correspond à l'intensité de l'onde lumineuse, dans la bande spectrale de mesure $\Delta_{mes}$, qui atteindrait le photodétecteur de mesure 20 en l'absence de gaz absorbant dans l'enceinte.

La comparaison entre $I(k)$ et $I_0(k)$, prenant la forme d'un ratio $\frac{I(k)}{I_0(k)}$, permet de définir une absorption $abs(k)$ générée par l'espèce gazeuse considérée à l'instant $k$.

Lors de chaque impulsion de la source de lumière 11, on peut ainsi déterminer $\mu(c_x(k))$, ce qui permet d'estimer $c_x(k)$ sachant que la relation entre $c_x(k)$ et $\mu(c_x(k))$ est connue.

L'expression (1) suppose une maîtrise de l'intensité $I_0(k)$ de l'onde lumineuse incidente 12 à l'instant de mesure $k$.

La figure 1B schématise un spectre d'émission d'une source de lumière 11 de type corps noir, obéissant à la loi de Planck :

$$L(\lambda, T) = \frac{2hc^2}{\lambda^5} \frac{1}{e^{\frac{hc}{\lambda K T}} - 1} \quad (2)$$

où

- $L(\lambda, T)$ est la luminance, dépendant de la longueur d'onde $\lambda$ et de la température de surface $T$ du corps noir;
- $h$ est la constante de Planck;
- $K$ est la constante de Boltzmann;
- $c$ est la vitesse de la lumière dans l'air.

Le spectre d'émission $S$ de la source de lumière 11 correspond à l'évolution de la luminance $L(\lambda, T)$ en fonction de $\lambda$, lorsque la source de lumière est portée à une température $T$. Généralement, la température $T$ est comprise entre 400°C et 800 °C.

On a représenté, sur la figure 1B, la bande spectrale d'illumination $\Delta_{12}$, de la source de lumière 11 s'étendant entre 1 $\mu$m et 10 $\mu$m. On a également représenté, par un trait en pointillés, la bande spectrale de référence $\Delta_{ref}$ ainsi que la bande spectrale de mesure $\Delta_{mes}$.

Ce type de source de lumière est particulièrement avantageux, car cela permet de moduler le spectre d'illumination $S$ par une simple modulation de la température $T$ de la source. Ainsi, à chaque température $T$ est associé un spectre d'illumination $S$.

Il est connu que l'émissivité d'une source de lumière de type corps noir ou corps gris, varie avec le temps, et peut notamment subir une décroissance résultant du vieillissement de la source de lumière. La variation temporelle de l'émission de la source de lumière 11 est prise en compte par le photodétecteur de référence $20_{ref}$. Ce dernier est agencé pour détecter une onde lumineuse de référence $12_{ref}$, représentative de l'onde lumineuse incidente 12 émise par la source de lumière 11. L'onde lumineuse de référence $12_{ref}$ atteint le photodétecteur de référence $20_{ref}$ sans interagir avec le gaz $G$, ou sans interagir significativement avec ce dernier.

L'intensité de l'onde lumineuse de référence $12_{ref}$, détectée par le photodétecteur de référence $20_{ref}$, à l'instant de mesure $k$, est désignée par le terme intensité de référence $I_{ref}(k)$. A partir de $I_{ref}(k)$, connaissant le spectre d'émission de la source de lumière 11, on peut estimer l'intensité $\hat{I}_0(k)$ de l'onde lumineuse qui atteindrait le photodétecteur de mesure 20 en l'absence de gaz $G$. L'intensité de référence peut également permettre de corriger l'intensité de mesure $I(k)$ pour prendre en compte le vieillissement de la source de lumière 11.

Le dispositif comporte un microprocesseur 30, relié à une mémoire 32 comportant des instructions permettant la mise en oeuvre des étapes de procédé décrites ci-dessous.

Selon un premier mode de réalisation, le microprocesseur 30 est configuré pour recevoir un signal représentatif de l'intensité $I_{ref}(k)$ de l'onde lumineuse de référence $12_{ref}$, mesurée par le photodétecteur de référence $20_{ref}$ à chaque instant de mesure $k$. Le microprocesseur 30 estime l'intensité $\hat{I}_0(k)$ à partir de $I_{ref}(k)$.

A partir de $I(k)$, on peut estimer l'absorption de l'onde lumineuse incidente selon l'expression :

$$abs(k) = 1 - \frac{I(k)}{\hat{I}_0(k)} \quad (3).$$

En utilisant l'expression (1), on obtient alors $\mu(c_x(k))$, puis $c_x(k)$.

Selon un deuxième mode de réalisation, le microprocesseur 30 est configuré pour recevoir un signal représentatif de l'intensité de référence $I_{ref}(k)$, puis pour effectuer une correction de l'intensité mesurée $I(k)$. L'intensité corrigée est notée *I*\*(*k*). Cette dernière correspond à l'intensité qui serait mesurée par le photodétecteur de mesure sans le vieillissement de la source de lumière. L'absorption *abs*(*k*) de l'onde lumineuse incidente peut alors être obtenue par l'expression :

$$abs(k) = 1 - \frac{I^*(k)}{I_0(k=0)} \quad (4),$$

où $I_0(k = 0)$ représente l'onde lumineuse incidente au photodétecteur de mesure, sans gaz absorbant dans l'enceinte, lors de l'instant de mesure initial $k = 0$, c'est-à-dire lorsque la source de lumière 11 est considérée comme neuve. En utilisant l'expression (1), on obtient alors $\mu(c_x(k))$, puis $c_x(k)$.

Il est usuellement considéré que le ratio entre l'émissivité de la source de lumière 11, respectivement dans la bande spectrale de référence $\Delta_{ref}$ et dans la bande spectrale de mesure $\Delta_{mes}$ décroît de la même façon. Selon une telle hypothèse :

- lorsqu'on met en oeuvre le premier mode de réalisation, l'intensité $\hat{I}_0(k)$ est simplement estimée à partir de $I_{ref}(k)$ à partir de la connaissance du spectre théorique d'émission de la source de lumière, ou selon une expression de type

$$\hat{I}_0(k) = I_{ref}(k) \frac{I_0(k=0)}{I_{ref}(k=0)} \ (5);$$

- lorsqu'on met en oeuvre le deuxième mode de réalisation, l'intensité corrigée $I^*(k)$ est obtenue par à partir de $I_{ref}(k)$, en appliquant une fonction de correction :

$$I^*(k) = \frac{I_{ref}(k=0)}{I_{ref}(k)} I(k) \ (6).$$

Cependant, l'inventeur a constaté que le vieillissement de la source de lumière 11 affecte différemment la bande spectrale de référence $\Delta_{ref}$ et la bande spectrale de mesure $\Delta_{mes}$. Contrairement à ce qui est suggéré dans le document FR3000548, le vieillissement dans la bande spectrale de mesure ne peut être considéré comme similaire au vieillissement dans la bande spectrale de référence.

L'inventeur a effectué un essai préliminaire, au cours duquel il a utilisé un capteur de mesure de test 20' et un capteur de référence de test 20'$_{ref}$ respectivement similaires aux capteurs de mesure et de référence décrits en lien avec la figure 1A. Durant la calibration, le gaz analysé était un gaz connu, en l'occurrence $CH_4$ dans de l'air ambiant, la concentration de $CH_4$ étant considérée comme nulle. Les paramètres expérimentaux étaient les suivants :

- Filtre de mesure 18 : filtre Heimann F3.25-180, centré sur une longueur d'onde de 3.25 $\mu$m, ce qui correspond à une longueur d'onde d'absorption de $CH_4$.
- Filtre de référence 18$_{ref}$ : filtre Heimann F3.91-90, centré sur une longueur d'onde de 3.91 $\mu$m.
- Photodétecteurs de mesure 20 et de référence 20$_{ref}$ : thermopile Heimann HCM Cx2 Fx.

Dans cet essai préliminaire, le filtre de mesure 18 définissait une bande spectrale de mesure $\Delta_{mes}$ centrée sur la longueur d'onde de volontairement étroite (3.25 $\mu$m), de façon à bien mettre en évidence le vieillissement observé par l'inventeur.

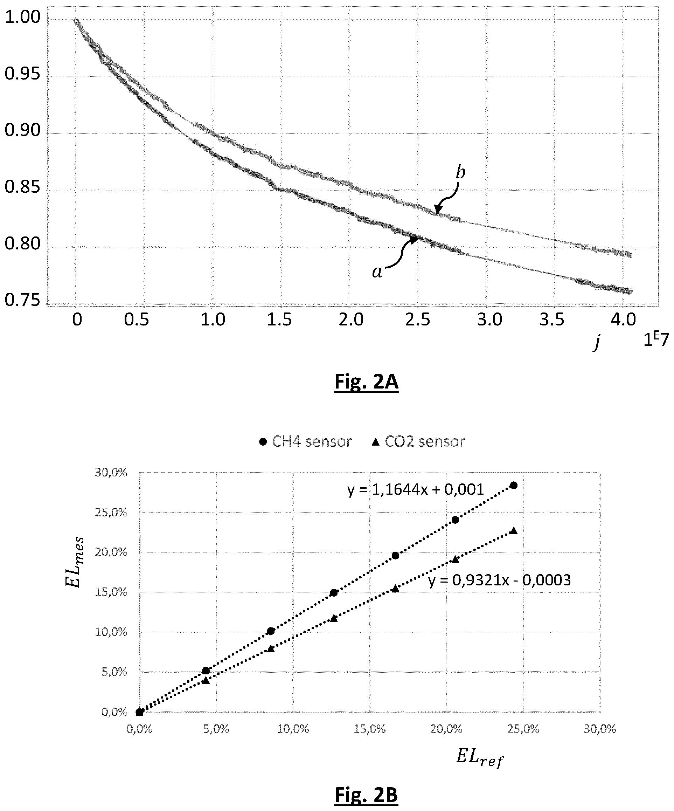

Une source de lumière de test 11', similaire à la source de lumière de décrite en lien avec la figure 1A, a été activée de façon impulsionnelle à différents instants *j*, entre un instant initial *j* = 0 et un instant final *j* = *J*. Chaque impulsion durait 60 ms, et était espacée de l'impulsion suivante par un intervalle temporel de 500 ms. Environ 40 millions d'impulsions ont été effectuées. La figure 2A représente les évolutions temporelles :

- d'une intensité de mesure *I*(*j*), mesurée par le photodétecteur de mesure de test 20' dans la bande spectrale de mesure $\Delta_{mes}$ (courbe a);
- de l'intensité de référence $I_{ref}(j)$, mesurée par le photodétecteur de référence de test 20'$_{ref}$ dans la bande spectrale de référence $\Delta_{ref}$ (courbe b).

Ces évolutions sont respectivement normalisées par l'intensité de mesure et l'intensité de référence à l'instant de calibration initial (*j* = 0).

Certaines parties de la courbe présentée sur la figure 2A ont été interpolées. On observe que sur la figure 2A, l'intensité de mesure $I(j)$ et l'intensité de référence $I_{ref}(j)$ décroissent avec le temps, ce qui était attendu. Cela correspond au vieillissement de la source de lumière 11. On observe également que les décroissances respectives dans la bande spectrale de mesure $\Delta_{mes}$ et dans la bande spectrale de référence $\Delta_{ref}$ sont différentes. Cela signifie que le vieillissement de la source de lumière 11 dans la bande spectrale de mesure $\Delta_{mes}$ est différent du vieillissement de la source de lumière 11 dans la bande spectrale de référence $\Delta_{ref}$. Ainsi, le ratio $\frac{I(j)}{I_{ref}(j)}$ varie en fonction du temps $j$. Cela signifie que le vieillissement de la source de lumière 11 s'accompagne d'une légère modification du spectre d'émission.

Un élément important de l'invention est que l'inventeur considère que le vieillissement de la source de lumière peut être assimilé à une variation de sa température, et plus précisément à une baisse de sa température. Lors de l'utilisation de la source de lumière, cette dernière est portée à une température nominale, par exemple égale à 870 K. L'inventeur a observé qu'en vieillissant, la source de lumière se comporte comme si sa température baissait, en dessous de la température nominale. La température nominale correspond aux premiers instants de fonctionnement de la source de lumière. Lorsque le nombre d'impulsions émises par la source de lumière augmente, le comportement de la source de lumière peut être modélisé en considérant que la température baisse progressivement, en dessous de la température nominale.

La source de lumière est considérée comme se comportant comme un corps noir. Dans l'expression (2), on observe que la luminance dépend à la fois de la température et de la longueur d'onde. L'inventeur a utilisé l'expression (2) en considérant différents niveaux de température, de façon à simuler l'effet d'une baisse de la température de la source de lumière sur l'intensité lumineuse émise par cette dernière, et cela dans différentes longueurs d'onde :

- à $\lambda$ = 3.91 $\mu$m, ce qui correspond à une bande spectrale de référence considérée comme non absorbée, ou de façon négligeable, par les espèces gazeuses les plus courantes ;
- à $\lambda$ = 3.25 $\mu$m, ce qui correspond à une bande spectrale d'absorption du méthane ($CH_4$);
- à $\lambda$ = 4.26 $\mu$m, ce qui correspond à une bande spectrale d'absorption du dioxyde de carbone ($CO_2$).

A partir d'un niveau de température nominale, égale à 870 K (Kelvin), l'inventeur a calculé, dans chacune des longueurs d'onde mentionnées ci-dessus, des pertes d'émissivité, notées EL, comme "Emissivity Loss".

Le tableau 1 regroupe des valeurs de perte d'émissivité $EL$ en fonction de la température de la source pour les trois longueurs d'onde sus-mentionnées. Chaque valeur de perte d'émissivité est calculée, à la longueur d'onde $\lambda$, et à la température $T$ selon l'expression :

$$EL(\lambda, T) = \frac{L(\lambda,T) - L(\lambda,T_0)}{L(\lambda,T_0)} \times 100 \quad (7)$$

, où $T_0$ correspond à la température nominale.

Tableau 1

| $T$ | $EL(\lambda = 3.25\ \mu m, T)\ (\%)$ | $EL(\lambda = 3.91\ \mu m, T)\ (\%)$ | $EL(\lambda = 4.26\ \mu m, T)\ (\%)$ |
|---|---|---|---|
| 870 (T = $T_0$) | 0.0 | 0.0 | 0.0 |
| 861.5 | 5.2 | 4.3 | 4.0 |
| 853 | 10.1 | 8.6 | 7.9 |
| 844.5 | 14.9 | 12.7 | 11.8 |
| 836 | 19.6 | 16.7 | 15.5 |
| 827.5 | 24.1 | 20.6 | 19.2 |
| 819 | 28.4 | 24.4 | 22.7 |

La figure 2B montre la perte d'émissivité $EL_{mes}$ dans la bande spectrale de mesure (axe des ordonnées) en fonction de la perte d'émissivité dans la bande spectrale de référence $EL_{ref}$ (axe des abscisses). La bande spectrale de mesure correspond soit à la longueur d'onde $\lambda$ = 3.25 $\mu$m (marques rondes sur la figure 2B), soit à la longueur d'onde $\lambda$ = 4.26 $\mu$m (marques triangles sur la figure 2B). La bande spectrale de référence correspond à la longueur d'onde $\lambda$

= 3.91 µm.

Suite à une interpolation linéaire des résultats, on observe que :

$$EL_{mes}\,(\lambda = 3.25\ \mu m) \simeq 1.1644\ EL_{ref} + 0.001 = h\big(EL_{ref}, \lambda_{mes}\big)\ (8)$$

et

$$EL_{mes}\,(\lambda = 4.26\ \mu m) \simeq 0.9321\ EL_{ref} + 0.0003 = h\big(EL_{ref}, \lambda_{mes}\big)\ (9)$$

La fonction *h* est une fonction du veillissement de la source dans la bande spectrale de mesure, à partir du vieillissement de la source dans la bande spectrale de référence. La fonction *h* est obtenue sur la base de calculs théoriques, en prenant en compte différentes températures de la source, en utilisant l'expression (2).

On observe que la perte d'émissivité, dans chaque bande spectrale de mesure, évolue linéairement par rapport à la perte d'émissivité dans la bande spectrale de référence. Par ailleurs, la pente de chaque droite est différente de 1, ce qui confirme le fait que l'intensité émise par la source de lumière évolue, dans le temps, de façon différente, aux longueurs d'onde considérées. En effet, si le vieillissement était identique à chaque longueur d'onde, comme considéré dans l'art antérieur, la pente de chaque droite serait égale à 1.

Suite aux calculs théoriques, ayant donné lieu aux résultats présentés sur le tableau 1 et représentés sur la figure 2B, l'inventeur a effectué des essais expérimentaux, en utilisant une source de lumière de test, un photodétecteur de mesure de test et un photodétecteur de référence de test, tels que précédemment décrits. Le photodétecteur de mesure de test a été associé à un filtre optique de mesure, centré sur la longueur d'onde de 3.25 µm.

Le photodétecteur de référence a été associé à un filtre optique de référence, centré sur la longueur d'onde de 3.91 µm.

Dans une première série d'essais, l'inventeur a effectué des essais de vieillissement à chaque longueur d'onde. La source de test a été disposée face à un photodétecteur de mesure de test, associé au filtre optique de mesure, ainsi que face à un photodétecteur de référence de test, associé au filtre optique de référence. Les essais de vieillissement visent à mesurer expérimentalement le vieillissement de la source de lumière, à chaque longueur d'onde. La source de mesure de test a été activée selon 18 millions d'impulsions successives, la durée de chaque impulsion étant de 60 ms, l'intervalle temporel entre deux impulsions successives s'élevant à 500 ms. Ainsi, la durée de cette première série d'essais de vieillissement était d'environ 104 jours.

Après j impulsions, on a mesuré les intensités I_j, I_{ref,j} respectivement mesurées par le photodétecteur de mesure de test et le photodétecteur de mesure de référence à des instants *j*. A partir de chaque mesure, on a calculé les pertes de signal détecté, représentatives des pertes d'émissivités de la source de lumière, et exprimées en %, à chaque longueur d'onde, selon les expressions :

$$EL_{ref}(j) = \frac{I_{ref}(j=0) - I_{ref}(j)}{I_{ref}(j=0)} \times 100\ \ (10)$$

et

$$EL_{mes}(j) = \frac{I(j=0) - I(j)}{I(j=0)} \times 100\ \ (11)$$

Les grandeurs $EL_{ref}(j)$ et $EL_{mes}(j)$, telles que définies dans les expressions (10) et (11), sont respectivement représentatives du vieillissement de la source de lumière dans la bande spectrale de référence et de mesure. Le ratio entre ces deux grandeurs correspond au vieillissement différentiel de la source de lumière, dans les bandes de spectrales de référence et de mesure.

Le tableau 2 rassemble les résultats de ces mesures.

Tableau 2

| Référence de l'instant de mesure (j) | $EL_{mes}(j)$ (%) | $EL_{ref}(j)$ (%) |
|---|---|---|
| 0 | 0.0 | 0.0 |

(suite)

| Référence de l'instant de mesure (j) | $EL_{mes}(j)$ (%) | $EL_{ref}(j)$ (%) |
|---|---|---|
| 2073600 | 7.6 | 6.3 |
| 4147200 | 10.2 | 8.7 |
| 6220800 | 13.2 | 11.3 |
| 9676800 | 18.5 | 16.0 |
| 11750400 | 20.2 | 17.4 |
| 17971200 | 24.0 | 20.9 |

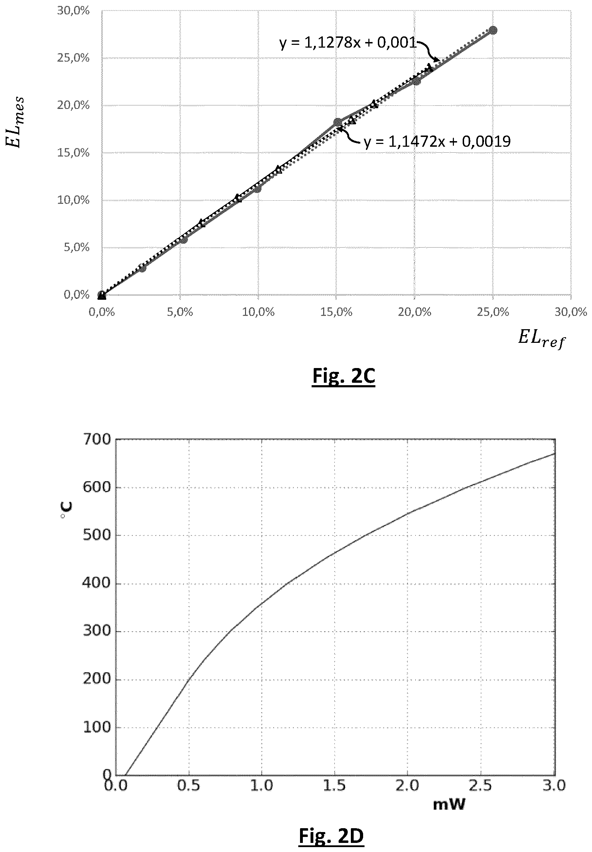

L'inventeur a ensuite effectué des simulations expérimentales de vieillissement d'une autre source de lumière de test. L'alimentation électrique de la source de lumière de test a été portée à différents niveaux de puissance, de façon à faire varier la température de la source. On est parti d'une puissance nominale de 2500 µW, correspondant à une température nominale de la source égale à 870 K. On a ensuite diminué progressivement la puissance du courant d'alimentation. A chaque niveau de puissance $P_j$, on a mesuré les intensités $I(P_j)$ et $I_{ref}(P_j)$ respectivement mesurées par un photodétecteur de mesure et un photodétecteur de référence de test. La figure 2D représente une fonction permettant de faire un lien avec la puissance du courant d'alimentation (axe des abscisses - mW) et la température de la source de lumière (axe des ordonnées, en °C).

A partir de chaque mesure, on a calculé les pertes de signal détecté, représentatives de la perte d'émissivité de la source de lumière, exprimées en %, à chaque longueur d'onde, selon les expressions :

$$EL_{ref}(j) = \frac{I_{ref}(P_{j=0}) - I_{ref}(P_j)}{I_{ref}(P_{j=0})} \times 100 \quad (12)$$

et

$$EL_{mes}(j) = \frac{I(P_{j=0}) - I(P_j)}{I(P_{j=0})} \times 100 \quad (13)$$

Le tableau 3 rassemble les résultats de ces mesures.

Tableau 3

| Puissance $P_j$ | Température (K) | $EL_{mes}(j)$ (%) | $EL_{ref}(j)$ (%) |
|---|---|---|---|
| 2500 ($P_0$) | 870 | 0.0 | 0.0 |
| 2450 | 863 | 2.8 | 2.6 |
| 2400 | 858 | 5.9 | 5.2 |
| 2300 | 843 | 11.2 | 9.9 |
| 2200 | 830 | 18.2 | 15.1 |
| 2100 | 815 | 22.6 | 20.1 |
| 2000 | 800 | 27.9 | 25 |

Les mesures présentées dans les tableaux 2 et 3 sont représentées sur la figure 2C. La figure 2C montre la perte relative de signal détecté $EL_{mes}$ dans la bande spectrale de mesure (axe des ordonnées) en fonction de la perte relative de signal détecté dans la bande spectrale de référence $EL_{ref}$ (axe des abscisses). La bande spectrale de mesure correspond à la longueur d'onde λ = 3.25 µm. La bande spectrale de référence correspond à la longueur d'onde λ = 3.91 µm. Sur la figure 2C, les marques carrées et rondes correspondent respectivement aux essais de simulation expérimentale de vieillissement (tableau 3) et aux essais de vieillissement réel (tableau 2).

Suite à une interpolation linéaire des résultats, on observe que :

- pour les résultats obtenus à partir des essais de vieillissement réel :

$$EL_{mes} \simeq 1.1472\ EL_{ref} + 0.0019 = f\left(EL_{ref}, \lambda_{mes}\right)\ (14),$$

ce qui correspond à la droite en pointillés blancs. La fonction *f* est une fonction du vieillissement de la source dans la bande spectrale de mesure, à partir du vieillissement de la source dans la bande spectrale de référence. La fonction *f* est obtenue par les essais de vieillissement réels.
- pour les résultats obtenus à partir des essais de simulation expérimentale de vieillissement :

$$EL_{mes} \simeq 1.1278\ EL_{ref} + 0.001 = g\left(EL_{ref}, \lambda_{mes}\right)\ (15),$$

ce qui correspond à la droite en pointillés noirs. La fonction *g* est une fonction du vieillissement de la source dans la bande spectrale de mesure, à partir du vieillissement de la source dans la bande spectrale de référence. La fonction *g* est obtenue par les essais de simulation du vieillissement par variation de la température de la source.

Les deux séries d'essais aboutissent à l'obtention d'une expression linéaire, reliant l'effet du vieillissement dans la bande spectrale de référence à l'effet du vieillissement dans la bande spectrale de mesure. On observe que les expressions (14) et (15) correspondent à des pentes comparables (respectivement 1.1472 et 1.1278), ce qui est également comparable à la pente résultant de l'expression (8), c'est-à-dire 1.1644.

Dans les essais qui précèdent, l'essai le plus représentatif du vieillissement de la source de lumière est probablement l'essai décrit en lien avec le tableau 2. On utilise alors une source de lumière de test durant une durée très longue, afin de mesurer expérimentalement le vieillissement différentiel dans la bande spectrale de référence et dans la bande spectrale de mesure. Le vieillissement différentiel peut notamment être exprimé sous la forme d'un ratio entre les grandeurs $EL_{mes}(j)$ et $EL_{ref}(j)$. Cela correspond à la pente de l'expression (14), soit 1.1472.

Cependant, un tel essai est long à mettre en oeuvre. Les résultats qui précèdent montrent que le vieillissement différentiel de la source de lumière peut faire l'objet d'une calibration en prenant en compte différents niveaux de température, ou différents niveaux de puissance d'alimentation, de la source de lumière. Il s'agit d'un élément important de l'invention : le vieillissement d'une source de lumière peut être appris en effectuant une calibration au cours de laquelle on prend en compte différents niveaux de température (ou différents niveaux du courant d'alimentation) de la source de lumière.

Selon une première possibilité, décrite en lien avec le tableau 1 et la figure 2B, on prend en compte une expression théorique de la luminance de la source, pour différentes températures, respectivement dans les bandes spectrales de mesure et de référence. On obtient ainsi un vieillissement différentiel théorique, représentant l'évolution relative de la quantité de lumière émise par la source de lumière dans les bandes spectrales de mesure et de référence. Aux longueurs d'onde considérées (3.25 $\mu$m et 3.91 $\mu$m), le vieillissement différentiel théorique peut être considéré, en première approche, comme la pente de l'expression (8), soit 1.1644. Selon cette première possibilité, connaissant vieillissement de la source de lumière dans la bande spectrale de référence, il est possible d'estimer le vieillissement de la source de lumière dans la bande spectrale de mesure, par l'application de la fonction *h* définie par l'expression (8).

Selon une deuxième possibilité, décrite en lien avec le tableau 3 et la figure 2C, on fait varier expérimentalement la température d'une source de lumière de test, par exemple en modifiant la puissance du courant d'alimentation. A différents niveaux de puissance (ou à différents niveaux de température), on mesure expérimentalement l'évolution relative de la quantité de lumière émise par la source de lumière dans les bandes spectrales de mesure et de référence. Aux longueurs d'onde considérées (3.25 $\mu$m et 3.91 $\mu$m), le vieillissement différentiel théorique peut être considéré, en première approche, comme la pente de l'expression (15), soit 1.1278. Selon cette deuxième possibilité, connaissant vieillissement de la source de lumière dans la bande spectrale de référence, il est possible d'estimer le vieillissement de la source de lumière dans la bande spectrale de mesure, par l'application de la fonction *g* définie par l'expression (15). Ce type de calibration expérimentale est facile et rapide à mettre en oeuvre. Lorsqu'on dispose de plusieurs dispositifs d'analyse de gaz, chaque dispositif étant constitué d'une source de lumière, d'un photodétecteur de mesure et d'un photodétecteur de référence, une telle calibration peut être effectuée sur la source de lumière de chaque dispositif, prise individuellement. Autrement dit, la source de lumière de test est confondue avec la source de lumière du dispositif. De façon alternative, une telle calibration peut être réalisée avec une source de lumière de test, considérée comme représentative de la source de lumière équipant le dispositif d'analyse.

La première possibilité est purement théorique. Elle ne prend en compte que le vieillissement de la source de lumière. La deuxième possibilité est plus expérimentale, et prend également en compte une évolution potentielle de la réponse des photodétecteurs, qu'il s'agisse du photodétecteur de mesure que du photodétecteur de référence.

Quelles que soient les possibilités, il est possible, à partir d'un vieillissement de la source de lumière mesuré dans la bande spectrale de référence, d'estimer un vieillissement de la source de lumière dans la bande spectrale de mesure. Le vieillissement de la source de lumière dans la bande spectrale de référence peut être estimé en comparant l'intensité lumineuse, dans la bande spectrale de référence, entre un instant initial et un instant de mesure. La comparaison peut par exemple prendre la forme d'une soustraction. La comparaison peut être normalisée par l'intensité lumineuse, dans la bande spectrale de référence, à l'instant initial, comme dans les expressions (10) ou (12). Cela permet d'obtenir un vieillissement compris entre 0 et 1.

Il résulte de ce qui précède qu'en prenant en compte, de façon théorique (première possibilité) ou expérimentale (deuxième possibilité), une variation de la température de la source de lumière, et plus précisément une diminution de la température de la source de lumière en dessous d'une température nominale, il est possible de déterminer une fonction de correction $\delta$ caractérisant la variation d'émissivité de la source de lumière relativement dans les deux bandes spectrales $\Delta_{mes}$ et $\Delta_{ref}$. Moyennant une mesure du vieillissement de la source de lumière dans la bande spectrale de référence, correspondant à la grandeur $EL_{ref}$, la fonction de correction $\delta$ permet d'estimer un vieillissement de la source de lumière dans la bande spectrale de mesure, correspondant à la grandeur $EL_{mes}$ telle que définie l'expression (11).

Lors du fonctionnement du détecteur, tel que décrit en lien avec l'expression (1), en chaque instant de mesure k, à partir :

- d'une mesure de l'intensité lumineuse de référence $I_{ref}(k = 0)$ à un instant initial;
- d'une mesure de l'intensité lumineuse de référence $I_{ref}(k)$ à un instant de mesure $k$ ;

la fonction de correction $\delta$, appliquée à l'intensité lumineuse de référence $I_{ref}(k)$, permet d'estimer l'intensité lumineuse $\widehat{I_0}(k)$, émise par la source de lumière, à l'instant $k$, dans la bande spectrale de mesure, en l'absence de gaz entre la source de lumière et le photodétecteur de mesure.

La fonction de correction peut être telle que :

$$\widehat{I_0}(k) = \delta\left(I_{ref}(k)\right) \quad (16)$$

En fonction de la calibration effectuée, la fonction de correction $\delta$ est telle que :

$$\widehat{I_0}(k) = \delta\left(I_{ref}(k)\right) = I_0(k = 0) \times \left[1 - \frac{h(EL_{ref}(k, \lambda_{mes}))}{100}\right] \quad (17)$$

Avec

$$EL_{ref}(k) = \frac{I_{ref}(k = 0) - I_{ref}(k)}{I_{ref}(k = 0)} \times 100 \quad (18)$$

La grandeur $I_0(k = 0)$ correspond à l'intensité, mesurée, par le photodétecteur de mesure, à l'instant initial $k = 0$, en l'absence de gaz entre le photodétecteur de mesure et la source de lumière. Elle peut être mesurée, par le photodétecteur de mesure, en l'absence de gaz entre la source de lumière et le photodétecteur de mesure. Elle peut également être estimée, à partir de $I_{ref}(k = 0)$, en connaissant la forme du spectre d'émission de la source de lumière, par exemple en utilisant l'expression (2).

La grandeur $I_{ref}(k = 0)$ correspond à l'intensité de référence mesurée à l'instant initial ($k=0$).

L'expression (17) suppose l'utilisation de la fonction d'estimation du vieillissement de la source $h$, déterminée de façon théorique, sur la base de l'expression (2), comme décrit en lien avec le tableau 1 et la figure 2B.

Il est possible d'utiliser une fonction du vieillissement de la source $g$, déterminée expérimentalement en prenant en compte différentes valeurs de température, comme décrit en lien avec le tableau 3 et la figure 2C. Dans ce cas, la fonction de correction $\delta$ est telle que :

$$\widehat{I_0}(k) = \delta\left(I_{ref}(k)\right) = I_0(k = 0) \times \left[1 - \frac{g(EL_{ref}(k, \lambda_{mes}))}{100}\right] \quad (19)$$

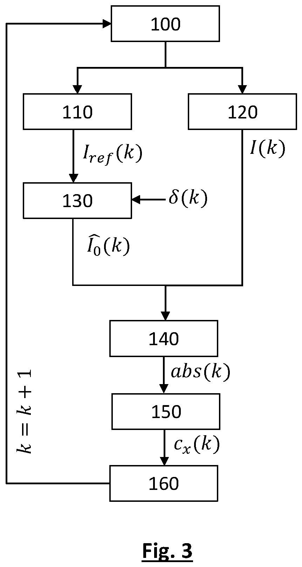

La figure 3 montre les principales étapes d'un procédé de mesure mettant en oeuvre l'invention.

Etape 100 : illumination du gaz à un instant $k$ ;

Etape 110 : mesure de l'intensité de référence $I_{ref}(k)$, dans la bande spectrale de référence $\Delta_{ref}$, par le photodétecteur de référence $20_{ref}$.

Etape 120 : mesure de l'intensité $I(k)$ du rayonnement 14 transmis par le gaz, dans la bande spectrale de mesure $\Delta_{mes}$, par le photodétecteur de mesure 20.

Etape 130 : estimation d'une intensité $\hat{I}_0(k)$ qui serait détectée par le photodétecteur de mesure 14, dans la bande spectrale de mesure $\Delta_{20}$, en l'absence de gaz dans l'enceinte. L'estimation est effectuée en prenant en compte la fonction de correction $\delta(k)$, et en appliquant l'expression :

$$\hat{I}_0(k) = \delta\big(I_{ref}(k)\big).$$

Etape 140 : estimation d'une absorption $abs(k) = 1 - \frac{I(k)}{\hat{I}_0(k)}$ dans la bande spectrale de mesure $\Delta_{mes}$.

Etape 150 : à partir de l'absorption, estimation d'une quantité $c_x(k)$ d'une espèce gazeuse $G_x$ à partir du ratio en appliquant l'expression (1).

Etape 160 : réitération des étapes 100 à 150, en incrémentant l'instant de mesure k, ou sortie de l'algorithme.

L'étape 130 suppose une détermination du vieillissement $EL_{ref}(k, \lambda_{mes})$ de la source, dans bande spectrale de référence $\Delta_{ref}$. Comme précédemment indiqué, le vieillissement, dans la bande spectrale de référence, correspond à une comparaison entre l'intensité de référence, à l'instant de mesure $k$, et une intensité de référence, à un instant initial.

L'inventeur a simulé des mesures obtenues en considérant que le gaz à analyser comporte une concentration constante de $CH_4$. Dans les mesures simulées, l'inventeur a établi une fonction de correction successivement basée sur :

- la fonction de vieillissement *f*, obtenue après des mesures expérimentales effectuées lors du suivi du vieillissement d'une source de lumière de test, telle que décrite en lien avec le tableau 2 ;
- la fonction de vieillissement *g*, obtenue après des mesures expérimentales effectuées en modifiant la température de fonctionnement d'une source de lumière de test, telle que décrit en lien avec le tableau 3 ;
- la fonction de vieillissement *h*, obtenue sur la base d'une expression théorique de la luminance de la source à différentes longueurs d'onde, d'une source de lumière de test, telle que décrite en lien avec le tableau 1 ;

Dans chaque cas de figure, on a estimé une erreur, en terme de ppm de $CH_4$, sans utilisation d'une fonction de correction, ainsi qu'en utilisant des fonctions de correction respectivement basées sur les fonctions *f, g* et *h*. L'erreur est déterminée en disposant un dispositif d'analyse de gaz dans de l'air ambiant, dans lequel la concentration en $CH_4$ est nulle. A partir de mesures de $I_{ref}$ et de $I_{mes}$, on obtient une concentration en $CH_4$ à partir de l'expression (1).

Le tableau 4 représente, en fonction de l'instant de mesure $k$, les erreurs obtenues. Dans le tableau 4, chaque instant de mesure correspond à une impulsion de la source de lumière. Il est rappelé que l'écart temporel entre deux impulsions successives est de 500 ms.

Tableau 4

| k | absence de correction | correction utilisant *f* | correction utilisant *g* | correction utilisant *h* |
|---|---|---|---|---|
| 0 | 0 | 0 | 0 | 0 |
| 2073600 | 7592 | 1472 | 2162 | 899 |
| 4147200 | 10411 | 1334 | 2304 | 556 |
| 6220800 | 14943 | 1630 | 2984 | 564 |
| 9676800 | 22597 | 681 | 2601 | -646 |
| 11750400 | 27564 | 1161 | 3424 | -364 |
| 17971200 | 36398 | 282 | 2936 | -1767 |

Par rapport à l'absence de correction, le recours à une fonction de correction, quelle qu'elle soit, permet de limiter significativement l'erreur de mesure.

La correction basée sur la fonction de vieillissement *f* est la plus performante. Il s'agit d'un résultat logique, puisque la fonction de vieillissement *f* est obtenue après un suivi du processus de suivi du vieillissement réel de la source de lumière. Cependant, on remarque que les erreurs commises à partir de corrections basées sur les fonctions de vieillissement *g* et *h* sont acceptables. Ainsi, le recours à de telles fonctions de vieillissement semble constituer une façon prometteuse pour corriger le vieillissement différentiel de la source de lumière. En effet, les fonctions de vieillissement *g* et *h* peuvent être rapidement obtenues, et ne nécessitent pas de suivre tout le processus de vieillissement d'une source de lumière. Elles forment un compromis particulièrement avantageux entre l'erreur de mesure et la facilité d'obtention de la fonction de correction.

Selon une variante, au cours de l'étape 130, on corrige la valeur *I(k)* de l'intensité mesurée par le capteur de mesure, à l'instant de mesure, en prenant en compte la fonction de correction $\delta$, en fonction de l'intensité de référence mesurée à l'instant de mesure On obtient une intensité corrigée *I\*(k)*. La fonction de correction $\delta$ peut être exprimée en utilisant la fonction de vieillissement correspondant à l'expression (9), de telle sorte que :

$$I^*(k) = \delta\big(I(k), I_{ref}(k)\big) = I(k) \times \left[1 - \frac{h(EL_{ref}(k, \lambda_{mes}))}{100}\right] \quad (20)$$

Avec

$$EL_{ref}(k) = \frac{I_{ref}(k=0) - I_{ref}(k)}{I_{ref}(k=0)} \times 100 \quad (21)$$

*I\*(k)* correspond à une valeur qui serait mesurée, par le photodétecteur de mesure, en l'absence de vieillissement de la source de lumière.

On comprend que dans l'expression (20), on peut utiliser la fonction *g* à la place de la fonction *h*.

Selon cette variante, au cours de l'étape 140, l'absorption est obtenue selon l'expression :

$$abs(k) = 1 - \frac{I^*(k)}{I_0(k=0)} \quad (22)$$

$I_0(k=0)$ correspond à une valeur d'intensité qui serait mesurée, par le photodétecteur de mesure, en l'absence de vieillissement de la source de lumière, et en l'absence de gaz entre la source de lumière et le photodétecteur de mesure.

L'invention pourra être mise en oeuvre pour la détection d'une quantité d'espèces gazeuses $G_x$ présentant des longueurs d'onde d'absorption comprises dans la bande spectrale de mesure $\Delta_{mes}$. Cette dernière peut être étroite, comme dans l'exemple expérimental décrit ci-avant. Elle peut également être large, de façon à inclure, par exemple, les bandes spectrales d'absorption $\Delta_x$ de plusieurs espèces gazeuses différentes.

**Revendications**

1. Procédé de mesure d'une quantité ($c_x$) d'une espèce gazeuse ($G_x$) présente dans un gaz, l'espèce gazeuse étant apte à absorber une lumière dans une bande spectrale d'absorption ($\Delta_x$) le procédé comportant les étapes suivantes :

   a) disposition du gaz entre une source de lumière (11) et un photodétecteur de mesure (20), la source de lumière (11) étant apte à émettre une onde lumineuse incidente (12), l'onde lumineuse incidente se propageant à travers le gaz vers le photodétecteur de mesure (20), la source de lumière étant parcourue par un courant électrique d'alimentation, de façon à porter la source de lumière à une valeur de température ;
   b) illumination du gaz (G) par la source de lumière (11) ;
   c) mesure, par le photodétecteur de mesure (20), d'une intensité (*I(k)*), dite intensité de mesure, d'une onde lumineuse (14) transmise par le gaz, dans une bande spectrale de mesure ($\Delta_{mes}$), comportant la bande spectrale d'absorption ($\Delta_x$);
   d) mesure, par un photodétecteur de référence ($20_{ref}$), d'une intensité ($I_{ref}(k)$), dite intensité de référence, d'une onde lumineuse ($12_{ref}$) de référence, l'onde lumineuse de référence étant émise par la source de lumière (11) dans une bande spectrale de référence ($\Delta_{ref}$);

   les étapes b) à d) étant mises en oeuvre à une pluralité d'instants de mesure (1 ... *k* ... *K*), le procédé comportant, à chaque instant de mesure :

   e) à partir de l'intensité de référence ($I_{ref}(k)$) mesurée par le photodétecteur de référence, prise en compte d'une fonction de correction ($\delta$), représentative d'une variation d'une intensité de l'onde lumineuse incidente (12) dans la bande spectrale de mesure ($\Delta_{mes}$) relativement à une intensité de l'onde lumineuse incidente (12) dans la bande spectrale de référence ($\Delta_{ref}$) ;

f) estimation d'une quantité ($c_x(k)$) de l'espèce gazeuse ($G_x$), à partir de l'intensité de mesure mesurée lors de l'étape c), de l'intensité de référence mesurée lors de l'étape d), et de la fonction de correction prise en compte lors de l'étape e) ;

le procédé étant **caractérisé en ce que** la fonction de correction ($\delta$) est préalablement établie au cours d'une phase de calibration, en comparant, à différents niveaux de température, ou à différents niveaux du courant d'alimentation, des intensités lumineuses respectivement émises, par une source de lumière de test, dans la bande spectrale de mesure et dans la bande spectrale de référence, la source de lumière de test étant considérée comme représentative de la source de lumière mise en oeuvre dans l'étape b), à chaque instant de mesure.

2. Procédé selon la revendication 1, dans lequel la calibration est réalisée en utilisant une expression théorique définissant une intensité d'émission de la source de lumière de test ($L(\lambda, T)$) en fonction de la température de la source de lumière ($T$) et de la longueur d'onde ($\lambda$), la calibration étant réalisée en considérant respectivement une longueur d'onde dans la bande spectrale de mesure ($\Delta_{mes}$) et une longueur d'onde dans la bande spectrale de référence ($\Delta_{ref}$).

3. Procédé selon la revendication 2, dans laquelle la calibration comporte une détermination d'une fonction de vieillissement ($h$), reliant un vieillissement de la source de lumière, dans la bande spectrale de référence, à un vieillissement de la source de lumière, dans la bande spectrale de mesure, le procédé étant tel que la fonction de correction ($\delta$) est déterminée à partir de la fonction de vieillissement ($h$).

4. Procédé selon la revendication 1, dans lequel la calibration est réalisée de façon expérimentale, et comporte :

- disposition de la source de lumière de test (11'), face à un photodétecteur de mesure de test (20'), et face à un photodétecteur de référence de test ($20'_{ref}$), le photodétecteur de mesure de test et le photodétecteur de référence de test étant respectivement représentatifs du photodétecteur de mesure (20) et du photodétecteur de référence ($20_{ref}$);
- illumination du photodétecteur de mesure de test et du photodétecteur de référence de test par la source de lumière de test, la source de lumière de test étant successivement portée à différents valeurs de température par différents courants d'alimentation;
- à chaque valeur de température, comparaison d'une intensité lumineuse ($I(j)$) détectée par le photodétecteur de mesure de test, dans la bande spectrale de mesure ($\Delta_{mes}$), avec une intensité lumineuse ($I_{ref}(j)$) détectée par le photodétecteur de référence de test, dans la bande spectrale de référence ($\Delta_{ref}$), la fonction de correction étant établie à partir des comparaisons effectuées à chaque valeur de température.

5. Procédé selon la revendication 4, dans laquelle la calibration comporte une détermination d'une fonction de vieillissement ($g$), reliant un vieillissement de la source de lumière, dans la bande spectrale de référence, à un vieillissement de la source de lumière, dans la bande spectrale de mesure, le procédé étant tel que la fonction de correction est déterminée à partir de la fonction de vieillissement.

6. Procédé selon la revendication 5, dans lequel la source de lumière de test (11') est confondue avec la source de lumière (11) mise en oeuvre dans l'étape b), à chaque instant de mesure.

7. Procédé selon l'une quelconque des revendications précédentes, dans lequel :

- l'étape e) comporte, à partir de la fonction de correction ($\delta$) et de l'intensité mesurée par le photodétecteur de référence ($I_{ref}(k)$) lors de l'étape d), une estimation d'une intensité ($\hat{I}_0(k)$) qui serait détectée, par le photodétecteur de mesure (20), en l'absence de gaz ;
- lors de l'étape f), la quantité ($c_x(k)$) d'espèce gazeuse est déterminée à partir d'une comparaison entre l'intensité mesurée par le photodétecteur de mesure, lors de l'étape c), et l'intensité ($\hat{I}_0(k)$) estimée lors de l'étape e).

8. Procédé selon l'une quelconque des revendications 1 à 6, dans lequel :

- l'étape e) comporte, à partir de la fonction de correction et de l'intensité mesurée par le photodétecteur de référence lors de l'étape d), une détermination d'une intensité corrigée ($I^*(k)$), l'intensité corrigée correspondant à une intensité qui serait détectée, par le photodétecteur de mesure (20), en l'absence de vieillissement de la source de lumière;

- lors de l'étape f), la quantité ($c_x(k)$) d'espèce gazeuse est déterminée à partir d'une comparaison entre l'intensité corrigée résultant de l'étape e) et d'une estimation d'une intensité ($I_0(k = 0)$) qui serait détectée par le photodétecteur de mesure, en l'absence de gaz entre la source de lumière et le photodétecteur de mesure, et en l'absence de vieillissement de la source de lumière.

9. Procédé selon l'une quelconque des revendications précédentes, dans lequel l'étape e) comporte, une estimation d'un vieillissement ($EL_{ref}(k)$) de la source de lumière, dans la bande spectrale de référence ($\Delta_{ref}$), à partir de l'intensité de référence mesurée à l'instant de mesure ($I_{ref}(k)$), et de l'intensité de référence mesurée à un instant initial ($I_{ref}(k = 0)$).

10. Procédé selon la revendication 9, dans lequel le vieillissement de la source de lumière, dans la bande spectrale de référence ($\Delta_{ref}$) est calculé à partir d'une comparaison entre l'intensité de référence mesurée à l'instant de mesure ($I_{ref}(k)$) et l'intensité de référence mesurée à l'instant initial ($I_{ref}(k = 0)$).

11. Dispositif (1) pour déterminer une quantité ($c_x(k)$) d'une espèce gazeuse ($G_x$) dans un gaz ($G$), le dispositif comportant :

- une source lumineuse (11) configurée pour émettre une onde lumineuse incidente (12) se propageant vers le gaz ($G$), l'onde lumineuse incidente s'étendant dans une bande spectrale d'absorption ($\Delta_x$) de l'espèce gazeuse ($G_x$) ;
- un photodétecteur de mesure (20), configuré pour détecter une onde lumineuse transmise (14) par le gaz, à différents instants de mesure ($k$), dans une bande spectrale de mesure et à en mesurer une intensité (($I(k)$)), dite intensité de mesure;
- un photodétecteur de référence ($20_{ref}$), configuré pour mesurer une intensité (($I_{ref}(k)$)), dite intensité de référence, d'une onde lumineuse de référence ($12_{ref}$) émise par la source de lumière (11), dans une bande spectrale de référence, aux différents instants de mesure ($k$);
- un processeur (30), programmé pour mettre en oeuvre les étapes e) et f) d'un procédé selon l'une quelconque des revendications précédentes, à partir de l'intensité de référence et de l'intensité de mesure,

le dispositif étant **caractérisé en ce que** le processeur est configuré pour mettre en oeuvre une fonction de correction établie au cours d'une phase de calibration, en comparant, à différents niveaux de température, ou à différents niveaux du courant d'alimentation, des intensités lumineuses respectivement émises, par une source de lumière de test, dans la bande spectrale de mesure et dans la bande spectrale de référence, la source de lumière de test étant considérée comme représentative de la source de lumière mise en oeuvre dans étape b) d'un procédé selon l'une quelconque des revendications précédentes, à chaque instant de mesure.


**Patentansprüche**

1. Verfahren zur Messung einer Menge ($c_x$) einer in einem Gas vorhandenen gasförmigen Spezies ($G_x$), wobei die gasförmige Spezies fähig ist, ein Licht in einem Absorptionsspektralband ($\Delta_x$) zu absorbieren, wobei das Verfahren die folgenden Schritte aufweist:

a) Anordnung des Gases zwischen einer Lichtquelle (11) und einem Messphotodetektor (20), wobei die Lichtquelle (11) fähig ist, eine einfallende Lichtwelle (12) zu emittieren, wobei die einfallende Lichtwelle sich durch das Gas zum Messphotodetektor (20) ausbreitet, wobei die Lichtquelle von einem elektrischen Speisestrom durchflossen wird, um die Lichtquelle auf einen Temperaturwert zu bringen;
b) Beleuchten des Gases ($G$) durch die Lichtquelle (11);
c) Messen, durch den Messphotodetektor (20), einer Messstärke genannten Stärke ($I(k)$) einer vom Gas übertragenen Lichtwelle (14) in einem Messspektralband ($\Delta_{mes}$), das das Absorptionsspektralband ($\Delta_x$) enthält;
d) Messen, durch einen Bezugsphotodetektor ($20_{ref}$), einer Bezugsstärke genannten Stärke ($I_{ref}(k)$) einer Bezugslichtwelle ($12_{ref}$), wobei die Bezugslichtwelle von der Lichtquelle (11) in einem Bezugsspektralband ($\Delta_{ref}$) emittiert wird;
wobei die Schritte b) bis d) zu einer Vielzahl von Messzeitpunkten (1 ... k ... K) durchgeführt werden,
wobei das Verfahren zu jedem Messzeitpunkt aufweist:

e) ausgehend von der vom Bezugsphotodetektor gemessenen Bezugsstärke ($I_{ref}(k)$), Berücksichtigung einer für eine Änderung einer Stärke der einfallenden Lichtwelle (12) im Messspektralband ($\Delta_{mes}$) bezüglich

einer Stärke der einfallenden Lichtwelle (12) im Bezugsspektralband ($\Delta_{ref}$) repräsentativen Korrekturfunktion ($\delta$);

f) Schätzung einer Menge ($c_x(k)$) der gasförmigen Spezies ($G_x$) ausgehend von der im Schritt c) gemessenen Messstärke, der im Schritt d) gemessenen Bezugsstärke, und der im Schritt e) berücksichtigten Korrekturfunktion;

wobei das Verfahren **dadurch gekennzeichnet ist, dass** die Korrekturfunktion ($\delta$) vorher während einer Kalibrierungsphase erstellt wird, indem auf unterschiedlichen Temperaturstufen oder auf unterschiedlichen Pegeln des Speisestroms Lichtstärken verglichen werden, die von einer Testlichtquelle im Messspektralband bzw. im Bezugsspektralband emittiert werden, wobei die Testlichtquelle als für die zu jedem Messzeitpunkt im Schritt b) verwendete Lichtquelle repräsentativ angesehen wird.

2. Verfahren nach Anspruch 1, wobei die Kalibrierung unter Verwendung eines theoretischen Ausdrucks durchgeführt wird, der eine Emissionsstärke ($L(\lambda,T)$) der Testlichtquelle abhängig von der Temperatur (T) der Lichtquelle und der Wellenlänge ($\lambda$) definiert, wobei die Kalibrierung unter Berücksichtigung einer Wellenlänge im Messspektralband ($\Delta_{mes}$) bzw. einer Wellenlänge im Bezugsspektralband ($\Delta_{ref}$) durchgeführt wird.

3. Verfahren nach Anspruch 2, wobei die Kalibrierung eine Bestimmung einer Alterungsfunktion (h) aufweist, die eine Alterung der Lichtquelle im Bezugsspektralband mit einer Alterung der Lichtquelle im Messspektralband verbindet, wobei das Verfahren so ist, dass die Korrekturfunktion ($\delta$) ausgehend von der Alterungsfunktion (h) bestimmt wird.

4. Verfahren nach Anspruch 1, wobei die Kalibrierung experimentell durchgeführt wird und aufweist:

- Anordnung der Testlichtquelle (11') gegenüber einem Test-Messphotodetektor (20') und gegenüber einem Test-Bezugsphotodetektor (20'$_{ref}$), wobei der Test-Messphotodetektor und der Test-Bezugsphotodetektor für den Messphotodetektor (20) bzw. den Bezugsphotodetektor (20$_{ref}$) repräsentativ sind;
- Beleuchtung des Test-Messphotodetektors und des Test-Bezugsphotodetektors durch die Testlichtquelle, wobei die Testlichtquelle nacheinander von verschiedenen Speiseströmen auf verschiedene Temperaturwerte gebracht wird;
- bei jedem Temperaturwert, Vergleich einer vom Test-Messphotodetektor im MessSpektralband ($\Delta_{mes}$) erkannten Lichtstärke (I(j)) mit einer vom Test-Bezugsphotodetektor im Bezugsspektralband ($\Delta_{ref}$) erkannten Lichtstärke ($I_{ref}(j)$), wobei die Korrekturfunktion ausgehend von den bei jedem Temperaturwert ausgeführten Vergleichen erstellt wird.

5. Verfahren nach Anspruch 4, wobei die Kalibrierung eine Bestimmung einer Alterungsfunktion (g) aufweist, die eine Alterung der Lichtquelle im Bezugsspektralband mit einer Alterung der Lichtquelle im Messspektralband verbindet, wobei das Verfahren so ist, dass die Korrekturfunktion ausgehend von der Alterungsfunktion bestimmt wird.

6. Verfahren nach Anspruch 5, wobei die Testlichtquelle (11') mit der zu jedem Messzeitpunkt im Schritt b) verwendeten Lichtquelle (11) zusammenfällt.

7. Verfahren nach einem der vorhergehenden Ansprüche, wobei :

- der Schritt e) ausgehend von der Korrekturfunktion ($\delta$) und der im Schritt d) vom Bezugsphotodetektor gemessenen Stärke ($I_{ref}(k)$) eine Schätzung einer Stärke ($\hat{I}_0(k)$) aufweist, die vom Messphotodetektor (20) in Abwesenheit von Gas erkannt würde ;
- im Schritt f) die Menge ($c_x(k)$) gasförmiger Spezies ausgehend von einem Vergleich zwischen der im Schritt c) vom Messphotodetektor gemessenen Stärke und der im Schritt e) geschätzten Stärke ($\hat{I}_0(k)$) bestimmt wird.

8. Verfahren nach einem der Ansprüche 1 bis 6, wobei :

- der Schritt e) ausgehend von der Korrekturfunktion und von der im Schritt d) vom Bezugsphotodetektor gemessenen Stärke eine Bestimmung einer korrigierten Stärke (I*(k)) aufweist, wobei die korrigierte Stärke einer Stärke entspricht, die vom Messphotodetektor (20) in Abwesenheit einer Alterung der Lichtquelle erkannt würde;
- im Schritt f) die Menge ($c_x(k)$) gasförmiger Spezies ausgehend von einem Vergleich zwischen der aus dem Schritt e) resultierenden korrigierten Stärke und einer Schätzung einer Stärke ($I_0(k = 0)$) bestimmt wird, die vom Messphotodetektor in Abwesenheit von Gas zwischen der Lichtquelle und dem Messphotodetektor und in Abwesenheit einer Alterung der Lichtquelle erkannt würde.

**9.** Verfahren nach einem der vorhergehenden Ansprüche, wobei der Schritt e) eine Schätzung einer Alterung ($EL_{ref}(k)$) der Lichtquelle im Bezugsspektralband ($\Delta_{ref}$) ausgehend von der zum Messzeitpunkt gemessenen Bezugsstärke ($I_{ref}(k)$) und der zu einem Ausgangszeitpunkt gemessenen Bezugsstärke ($I_{ref}(k = 0)$ aufweist.

**10.** Verfahren nach Anspruch 9, wobei die Alterung der Lichtquelle im Bezugsspektralband ($\Delta_{ref}$) ausgehend von einem Vergleich zwischen der zum Messzeitpunkt gemessenen Bezugsstärke ($I_{ref}(k)$) und der zum Ausgangszeitpunkt gemessenen Bezugsstärke ($I_{ref}(k = 0)$) berechnet wird.

**11.** Vorrichtung (1) zur Bestimmung einer Menge ($c_x(k)$) einer gasförmigen Spezies ($G_x$) in einem Gas (G), wobei die Vorrichtung aufweist:

- eine Lichtquelle (11), die konfiguriert ist, eine einfallende Lichtwelle (12) zu emittieren, die sich zum Gas (G) ausbreitet, wobei die einfallende Lichtwelle sich in einem Absorptionsspektralband ($\Delta_x$) der gasförmigen Spezies ($G_x$) erstreckt;
- einen Messphotodetektor (20), der konfiguriert ist, zu verschiedenen Messzeitpunkten (k) eine vom Gas übertragene Lichtwelle (14) in einem Messspektralband zu erkennen und von ihr eine Messstärke genannte Stärke (($I(k)$) zu messen;
- einen Bezugsphotodetektor ($20_{ref}$), der konfiguriert ist, zu den verschiedenen Messzeitpunkten (k) eine Bezugsstärke genannte Stärke (($I_{ref}(k)$) einer von der Lichtquelle (11) in einem Bezugsspektralband emittierten Bezugslichtwelle ($12_{ref}$) zu messen;
- einen Prozessor (30), der programmiert ist, die Schritte e) und f) eines Verfahrens nach einem der vorhergehenden Ansprüche ausgehend von der Bezugsstärke und der Messstärke durchzuführen,

wobei die Vorrichtung **dadurch gekennzeichnet ist, dass** der Prozessor konfiguriert ist, eine Korrekturfunktion anzuwenden, die während einer Kalibrierungsphase erstellt wird, indem auf verschiedenen Temperaturpegeln oder auf verschiedenen Pegeln des Speisestroms Lichtstärken verglichen werden, die von einer Testlichtquelle im Messspektralband bzw. im Bezugsspektralband emittiert werden, wobei die Testlichtquelle als für die zu jedem Messzeitpunkt im Schritt b) eines Verfahrens nach einem der vorhergehenden Ansprüche angewendete Lichtquelle repräsentativ angesehen wird.

**Claims**

**1.** Method for measuring an amount ($c_x$) of a gas species ($G_x$) present in a gas, the gas species being able to absorb light in an absorption spectral band ($\Delta_x$), the method comprising the following steps:

a) placing the gas between a light source (11) and a measurement photodetector (20), the light source (11) being able to emit an incident light wave (12), the incident light wave propagating through the gas to the measurement photodetector (20), the light source being passed through by an electrical supply current, so as to bring the light source to a temperature value;
b) illuminating the gas (G) with the light source (11);
c) measuring, with the measurement photodetector (20), an intensity ($I(k)$), referred to as the measurement intensity, of a light wave (14) transmitted by the gas, in a measurement spectral band ($\Delta_{mes}$) comprising the absorption spectral band ($\Delta_x$);
d) measuring, with a reference photodetector ($20_{ref}$), an intensity ($I_{ref}(k)$), referred to as the reference intensity, of a reference light wave ($12_{ref}$), the reference light wave being emitted by the light source (11) in a reference spectral band ($\Delta_{ref}$);

steps b) to d) being implemented at a plurality of measurement times (1 ... k ... K), the method comprising, at each measurement time:

e) on the basis of the reference intensity ($I_{ref}(k)$) measured by the reference photodetector, taking into account a correction function ($\delta$) representative of a variation in an intensity of the incident light wave (12) in the measurement spectral band ($\Delta_{mes}$) relative to an intensity of the incident light wave (12) in the reference spectral band ($\Delta_{ref}$);
f) estimating an amount ($c_x(k)$) of the gas species ($G_x$), on the basis of the measurement intensity measured in step c), of the reference intensity measured in step d), and of the correction function taken into account in step e);

the method being **characterized in that** the correction function ($\delta$) is established beforehand in a calibrating phase, by comparing, at various temperature levels, or at various supply-current levels, the light intensities emitted, by a test light source, in the measurement spectral band and in the reference spectral band, respectively, the test light source being considered to be representative of the light source employed in step b), at each measurement time.

2. Method according to Claim 1, wherein the calibration is carried out using a theoretical expression that defines an emission intensity ($L(\lambda, T)$) of the test light source as a function of the temperature ($T$) of the light source and of wavelength ($\lambda$), the calibration being carried out considering a wavelength in the measurement spectral band ($\Delta_{mes}$) and a wavelength in the reference spectral band ($\Delta_{ref}$), respectively.

3. Method according to Claim 2, wherein the calibration comprises determining an ageing function ($h$), relating an ageing of the light source, in the reference spectral band, to an ageing of the light source, in the measurement spectral band, the method being such that the correction function ($\delta$) is determined on the basis of the ageing function ($h$).

4. Method according to Claim 1, wherein the calibration is carried out experimentally, and comprises:

   - placing the test light source (11') facing a test measurement photodetector (20') and facing a test reference photodetector (20'$_{ref}$), the test measurement photodetector and the test reference photodetector being representative of the measurement photodetector (20) and of the reference photodetector (20$_{ref}$), respectively;
   - illuminating the test measurement photodetector and the test reference photodetector with the test light source, the test light source being successively brought to various temperature values by various supply currents;
   - at each temperature value, comparing a light intensity ($I(j)$) detected by the test measurement photodetector, in the measurement spectral band ($\Delta_{mes}$), with a light intensity ($I_{ref}(j)$) detected by the test reference photodetector, in the reference spectral band ($\Delta_{ref}$), the correction function being established on the basis of the comparisons made at each temperature value.

5. Method according to Claim 4, wherein the calibration comprises determining an ageing function ($g$), relating ageing of the light source, in the reference spectral band, to ageing of the light source, in the measurement spectral band, the method being such that the correction function is determined on the basis of the ageing function.

6. Method according to Claim 5, wherein the test light source (11') is none other than the light source (11) employed in step b), at each measurement time.

7. Method according to any one of the preceding claims, wherein:

   - step e) comprises, on the basis of the correction function ($\delta$) and of the intensity ($I_{ref}(k)$) measured by the reference photodetector in step d), estimating an intensity ($\hat{I}_0(k)$) that would be detected, by the measurement photodetector (20), in the absence of gas;
   - in step f), the amount ($c_x(k)$) of gas species is determined on the basis of a comparison between the intensity measured by the measurement photodetector, in step c), and the intensity ($\hat{I}_0(k)$) estimated in step e).

8. Method according to any one of Claims 1 to 6, wherein:

   - step e) comprises, on the basis of the correction function and of the intensity measured by the reference photodetector in step d), determining a corrected intensity ($I^*(k)$), the corrected intensity corresponding to an intensity that would be detected, by the measurement photodetector (20), in the absence of ageing of the light source;
   - in step f), the amount ($c_x(k)$) of gas species is determined on the basis of a comparison between the corrected intensity resulting from step e) and an estimate of an intensity ($I_0(k = 0)$) that would be detected by the measurement photodetector, in the absence of gas between the light source and the measurement photodetector, and in the absence of ageing of the light source.

9. Method according to any one of the preceding claims, wherein step e) comprises estimating an ageing ($EL_{ref}(k)$) of the light source, in the reference spectral band ($\Delta_{ref}$), on the basis of the reference intensity ($I_{ref}(k)$) measured at the measurement time, and of the reference intensity ($I_{ref}(k = 0)$) measured at an initial time.

10. Method according to Claim 9, wherein the ageing of the light source, in the reference spectral band ($\Delta_{ref}$), is computed

on the basis of a comparison between the reference intensity ($I_{ref}(k)$) measured at the measurement time, and the reference intensity ($I_{ref}(k = 0)$) measured at the initial time.

11. Device (1) for determining an amount ($c_x(k)$) of a gas species ($G_x$) in a gas ($G$), the device comprising:

   - a light source (11) configured to emit an incident light wave (12) that propagates to the gas ($G$), the incident light wave lying in an absorption spectral band ($\Delta_x$) of the gas species ($G_x$);
   - a measurement photodetector (20), configured to detect a light wave (14) transmitted by the gas, at various measurement times ($k$), in a measurement spectral band, and to measure an intensity (($I(k)$), referred to as the measurement intensity, thereof;
   - a reference photodetector ($20_{ref}$), configured to measure an intensity (($I_{ref}(k)$), referred to as the reference intensity, of a reference light wave ($12_{ref}$) emitted by the light source (11), in a reference spectral band, at the various measurement times ($k$);
   - a processor (30), programmed to implement steps e) and f) of a method according to any one of the preceding claims, on the basis of the reference intensity and of the measurement intensity,

   the device being **characterized in that** the processor is configured to implement a correction function established in a calibrating phase, by comparing, at various temperature levels, or at various supply-current levels, the light intensities emitted, by a test light source, in the measurement spectral band and in the reference spectral band, respectively, the test light source being considered to be representative of the light source employed in step b) of a method according to any one of the preceding claims, at each measurement time.

**Fig. 1A**

**Fig. 1B**

**Fig. 2A**

**Fig. 2B**

**Fig. 2C**

**Fig. 2D**

```
        ┌──────────┐
   ┌───▶│   100    │
   │    └──────────┘
   │      │      │
   │      ▼      ▼
   │  ┌──────┐ ┌──────┐
   │  │ 110  │ │ 120  │
   │  └──────┘ └──────┘
   │      │        │
   │   I_ref(k)   I(k)
   │      ▼
   │  ┌──────┐◀── δ(k)
   │  │ 130  │
   │  └──────┘
   │      │
   │    Î₀(k)
   │      ▼
   │  ┌──────┐
   │  │ 140  │
   │  └──────┘
   │      │ abs(k)
   │      ▼
   │  ┌──────┐
   │  │ 150  │
   │  └──────┘
   │      │ c_x(k)
   │      ▼
   │  ┌──────┐
   └──│ 160  │
      └──────┘
```

$I_{ref}(k)$   $I(k)$   $\delta(k)$   $\widehat{I_0}(k)$   $abs(k)$   $c_x(k)$   $k = k + 1$

**Fig. 3**

24

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5026992 A
- WO 2007064370 A
- US 20110042570 A
- FR 3000548
- US 2015338339 A1
- US 619142181 B
- FR 3077387 A1